Europäisches Patentamt

European Patent Office

Office européen des brevets

(19)

(11) Publication number: 0 361 957
A1

# (12) EUROPEAN PATENT APPLICATION

(21) Application number: 89309972.1

(22) Date of filing: 29.09.89

(51) Int. Cl.⁵: A61L 27/00 , A61L 15/16 , C12N 5/00

(30) Priority: 30.09.88 US 252249

(43) Date of publication of application:
04.04.90 Bulletin 90/14

(84) Designated Contracting States:
AT BE CH DE FR GB IT LI NL SE

(71) Applicant: ORGANOGENESIS INC.
83, Roger Street
Cambridge, MA 02142(US)

(72) Inventor: Bell, Eugene
305 Commonwealth Avenue
Boston Massachusetts 02215(US)
Inventor: Kagan, David T.
2 Washburn Place
Brookline Massachusetts 02146(US)

(74) Representative: Davies, Jonathan Mark et al
Reddie & Grose 16 Theobalds Road
London WC1X 8PL(GB)

(54) Tissue equivalents and methods of preparation thereof.

(57) The present invention provides tissue equivalents which have cell types differentiated from progenitor cells without exogenous chemical induction, and methods of making and using such tissue equivalents. The present invention also provides methods for differentiating progenitor cells without exogenous chemical induction.

EP 0 361 957 A1

## TISSUE EQUIVALENTS AND METHODS OF PREPARATION THEROF

### FIELD OF THE INVENTION

This invention relates to tissue equivalents which comprise cells produced by the differentiation of progenitor cells in the tissue equivalent lattice, without exogenous chemical induction of such differentiation, and to methods of preparing and using such tissue equivalents, including augmentation, repair or replacement of tissue in a living host. This invention further relates to methods for differentiating progenitor cells without exogenous chemical induction.

Tissue equivalents prepared from a hydrated collagen lattice contracted by a contractile agent, such as fibroblast cells or blood platelets, to form the tissue equivalent are disclosed in U.S. Patent Nos. 4,485,096; 4,485,097; 4,539,716; 4,546,500; 4,604,346; 4,835,102; and 4,837,379, all of which are incorporated herein by reference (hereinafter collectively referred to as "the Patents"). These tissue equivalents include, but are not limited to, equivalents of epithelial tissue, connective tissue, cartilage, bone, blood, organs, glands and blood vessels and comprise living cells and extracellular matrix molecules, principally collagen, and may optionally be provided with components not typically found in normal tissue. Various cell types can be grown in or on such tissue equivalents, including abnormal or transformed cells.

The aforementioned tissue equivalents are populated with cells that can remain alive for long periods of time and can be produced in quantity with the assurance that the units produced will be essentially uniform. Cells in such tissue equivalents resemble cells of normal tissue in their structural arrangement, in their biosynthetic output, and in their permeability. It should be understood that these tissue equivalents need not be human but may be those of any animal as desired.

Such tissue equivalents have a broad range of applications including applications in medical, pharmaceutical, cosmetic, industrial and other research and development; in testing product safety and in product development; and in tissue augmentation, repair and replacement and organ replacement.

In some tissue augmentation applications, the tissue equivalents are preformed and, then, surgically implanted at the site to be augmented. In yet other applications, the components of the tissue equivalent, i.e., the tissue precursor mixture, are mixed under conditions which prevent gelling of the tissue equivalent lattice and, then, injected at the site to be augmented under conditions which permit gelling in the host.

Animal model experiments involving injection of tissue precursor mixtures have shown that once in contact with host tissue, the mixture gels and is space filling. Gelling occurs, for example, as soon as the temperature of the precooled mixture is caused to rise by the surrounding higher temperature of the host. By injecting tissue precursor under the skin of the scalp, for, example, the space filling properties of the tissue that forms have been studied by gross observations and histologically by taking biopsies. The injected tissue precursor was seen to form into a mass of tissue that becomes vascularized within three to seven days and persists for long periods (months).

Experiments carried out in rats demonstrate that tissue equivalents made in accordance with the Patents, show excellent implantability. Lattices were implanted to the nape of the neck and maintained for 6-8 weeks. These were then recovered and examined. A high degree of vascularization had taken place, a key event in the ability of implants of tissue equivalents containing certain functional cells, for example, adipocytes, to take.

Certain types of cells that are derived from biopsies and are useful in producing tissue equivalents dedifferentiate when cultivated in vitro. These cells can be thought of as progenitor cells because, under appropriate circumstances, they can be caused to differentiate and provide progeny of a stable differentiated phenotype.

Progenitor cells may be obtained by dedifferentiating a given cell type. See, e.g., Negrel et al., Methods in Enzymology, 109:377-385 (Academic Press, Inc. 1985), Van and Roncari, Cell Tiss. Res. 195:317-329 (1978). Reported methods of differentiating progenitor cells typically involve the use of inducing agents. Dexamethasone, methyl isobutylxanthine, and 5-azacytidine, for example, have been used. For certain applications, e.g., fabrication of tissue-equivalents, it may be undesirable to use exogenous chemical induction. Thus, ways of differentiating progenitor cells without use of such agents is desired.

### SUMMARY OF THE INVENTION

The present invention provides methods of producing tissue equivalents having cell types which result from differentiation of progenitor cells without exogenous chemical induction. Exogenous chemical induction of the differentiation of progenitor cells is undesirable for certain applications. Inducing agents such as dexamethasone, for example, adversely affect the ability of a forming tissue equivalent to contract. Furthermore, such inducing agents may interfere with cell function.

One method of preparing tissue equivalents in accordance with the present invention comprises:

a. constituting a tissue precursor mixture, the mixture comprising a collagen solution, at least one contractile agent and at least one type of progenitor cell, under conditions to form a gel having the contractile agent and progenitor cells dispersed within the gel; and then

b. maintaining the gel prepared in step (a) under conditions which permit (i) the contraction of the gel to form a tissue equivalent and (ii) the differentiation without exogenous chemical induction of the progenitor cells.

Progenitor cells for use in the present invention include preadipocytes, undifferentiated embryonic cells, undifferentiated fetal cells, tissue cells dedifferentiated during cell culture, or organ cells dedifferentiated during cell culture.

The present invention also provides compositions and methods of augmenting, replacing and repairing tissue in a host. In some embodiments, tissue equivalents prepared as described above may be implanted at the site to be augmented. In yet other embodiments of the present invention, a tissue equivalent may be formed in situ. One method of augmenting tissue in a living host in accordance with the present invention comprises:

a. administering at the site to be augmented a tissue precursor mixture free of exogenous chemical inducing agents, the mixture comprising a collagen solution, at least one contractile agent and at least one type of progenitor cell, under conditions to form a gel having the contractile agent and progenitor cells dispersed within the gel mixture.

In a preferred embodiment of the present invention the progenitor cell is a preadipocyte. Tissue equivalents having predictable numbers of adipocytes were heretofore difficult to achieve. Control over adipocyte content in tissue equivalents provides, for example, tissue equivalents that more closely resemble their natural counterparts.

The present invention also provides methods of differentiating progenitor without exogenous chemical induction. One such method comprises:

a. constituting a mixture, the mixture comprising a collagen solution, at least one contractile agent and at least one type of progenitor cell, under conditions to form a gel having the contractile agent and progenitor cells dispersed within the gel mixture; and then

b. maintaining the gel mixture prepared in step (a) under conditions which permit (i) the contraction of the gel mixture, and (ii) differentiation, without exogenous chemical induction, of the progenitor cells.

DETAILED DESCRIPTION OF THE INVENTION

Tissue equivalents prepared from a hydrated collagen lattice contracted by a contractile agent to form the tissue equivalent are disclosed in the Patents. It has been unexpectedly discovered that tissue equivalents may be produced by the differentiation of tissue progenitor cells in the tissue equivalent lattice without exogenous chemical induction of such differentiation. Thus, the tissue equivalents of the present invention, although similar in both methods of preparation and use to those disclosed in the Patents, further comprise cells which have differentiated in the tissue equivalent without exogenous chemical induction.

As used herein: (i) "exogenous chemical induction" shall mean causing a progenitor cell to differentiate in a tissue equivalent by the addition, before, during or after formation of the tissue equivalent, of one or more agents which induce differentiation of progenitor cells, such agents include dexamethasone, methyl isobutylxanthine, indomethacin and 5-azacytidine; (ii) "progenitor cell" shall mean a cell which is capable of differentiating into another cell type; and (iii) "dedifferentiation" shall mean a process by which a cell loses certain characteristic morphological and/or biochemical properties.

Although exogenous chemical induction may, in some instances, increase the percentage of progenitor cells differentiating in a tissue equivalent, it may have undesirable side effects. For example, dexamethasone greatly reduces the capacity of a forming tissue equivalent to contract (Coulomb et al, J. Invest. Dermatol. 82:341-344, 1984). The advantages of obtaining differentiation of progenitor cells without exogenous chemical induction include, therefore, tissue formation and differentiation and function of other cell types present in or on the tissue equivalent without interference by such inducer chemicals.

Tissue equivalents of the present invention are prepared as described in the Patents, except that in accordance with the present invention, one or more progenitor cell types are additionally incorporated in or added to the tissue equivalent during formation thereof and subsequently differentiated in the tissue equivalent without exogenous chemical induction.

It is expected that many types of progenitor cells may be caused to differentiate in tissue-equivalents without the need for exogenous chemical induction. Such progenitor cells include many types of fetal and embryonic cells.

Tissue equivalents may be formed in accordance with the present invention by the differentiation without exogenous chemical induction of, for example, preadipocytes into adipocytes, in the tissue-equivalent lattice. This method enables the production of tissue equivalents containing predictable numbers of functional adipocytes heretofore difficult to achieve.

Tissue equivalents having predictable number of functional adipocytes are useful in preparing reconstructive tissue- equivalents and are particularly useful in the augmentation, replacement or reconstruction of breast tissue, connective tissue and other tissues generally in living hosts and are sometimes referred to hereinafter as "filler tissue equivalents."

Tissue equivalents prepared in accordance with the present invention using mixtures of non-inducible fibroblasts and progenitor cells were found to have relative proportions of adipocytes to fibroblasts that reflect the starting proportions. Control over adipocyte content in tissue equivalents is important because it enables construction of a tissue equivalent which mimics its natural counterpart. Adipocytes are important in determining tissue consistency or softness, for example, in breast tissue and some types of connective tissue.

Preadipocytes for use in the present invention may be obtained from adipocytes isolated from yellow fat, e.g., superadrenal fat. The adipocytes are dissociated from the fat, recovered, plated, and passaged. The resultant cells are dedifferentiated, i.e., have lost all lipid droplets, are firbroblast-like, and are hereinafter referred to as predipocytes. If desired, the preadipocytes may be cloned to select for phenotypic consistency. Preadipocytes or clones thereof which produce the adipocyte phenotype when induced to differentiate by exogenous chemical induction are selected for fabrication of tissue equivalents in accordance with the present invention.

Selected preadipocytes, both uncloned and cloned, were cultured in tissue culture ("TC") flasks, or were incorporated in tissue equivalents made in accordance with the Patents. In some cases, exogenous chemical induction of differentiation was induced over a period of 48 hours with dexamethasone 0.25uM and methyl isobutylxanthine 0.5mM.

After seven days, preadipocytes on plates which had been chemically induced were seen to contain visible refractile perinuclear droplets whereas uninduced preadipocytes on plates did not express the adipocyte phenotype. In contrast, within two weeks preadipocytes which were incorporated in tissue equivalents differentiated into fat cells containing stainable lipids whether or not they were exogenously induced with chemicals. Exogenous chemical induction of differentiation of progenitor cells in the tissue equivalent was found, in certain instances, to increase the number of cells expressing the adipocyte phenotype by 28%.

It was observed that preadipocytes cultured on plates detached as they differentiated and enlarged, whereas the differentiated cells in tissue equivalents remained anchored and were stellate in morphology. The capacity of cells to remain attached to the matrix is critical for tissue maturation and continued cell differentiation into fat cells.

The invention will be further understood with reference to the following examples, which are purely exemplary in nature, and are not meant to be utilized to limit the scope of the invention.

Materials used in the following examples were obtained from the sources indicated in the examples or made in accordance with the indicated publications.

Example 1:

Isolation of Adipocytes from the Float and Dedifferentation

Human yellow fat was minced and digested in collagenase Img/ml in DMEM. The digests were carried out to completion at 35°C on a shaker platform. The digest was run through a 500u polypropylene mesh, to remove large particles, and centrifuged at room temperature. The supernatant obtained is hereinafter referred to as the "the float."

The float (composed of adipocytes and oil released from adipocytes) was transferred to another vessel and processed separately from the pellet. The float was washed and the oil (top) discarded. The cells (5 ml of float) were mixed with 5 ml of medium DMEM-F12 + 10%CS and cultured in TC flasks. After several days, cells with lipid inclusions could be seen attached to the surface of the flask. The lipid inclusions were lost over time as the cells dedifferentiated and adopted a fibroblastic morphology.

Example 2:

Preparation of Preadipocytes from the Pellet

The pellet obtained from the centrifugation step in Example 1 above was washed and, if very bloody, centrifuged against a ficoll-hypaque gradient. The cells (composed of stromal fibroblasts and preadipocytes) were cultured in TC flasks in DMEM-F12 + 10%CS.

Example 3:

## Differentiation of Preadipocytes in a Tissue Equivalent

Tissue equivalents were prepared in accordance with the Patents.

Typically, an acid soluble rat tail collagen or acid soluble bovine tendon collagen was used. A premix, typically containing 10X MEM Earles salts (2.43ml), glutamine 200mM (0.24ml), gentamicin S 50mg/ml (0.03ml), bovine serum (2.7ml), $NaHCO_3$ 3.56%: NaOH 50mM (1.5ml), and bovine tendon collagen 1mg/ml in 0.05% acetic acid (20ml), was prepared and maintained chilled. A mixture of fibroblasts (human dermal fibroblasts (HDF) isolated from foreskin) and/or preadipocytes (PA)), obtained in accordance with Example 2 above was maintained in a separate solution cold (HDF and/or PA 250,000 cells/ml (3ml)). Before casting the tissue equivalent, the premeasured solutions were mixed together to constitute a tissue precursor mixture and transferred to sterile petri dishes and allowed to gel at 37°C and allowed to form a tissue equivalent.

After two days the preadipocytes in some tissue equivalents were induced to differentiate with a mixture of dexamethasone 0.25uM and isobutylmethylxanthine 0.5mm. After 48 hours this cocktail was removed and the lattices were cultured in DMEM-F12 + 10%CS. Within a week cells could be seen with perinuclear lipid inclusions. Over the course of the next week many of the lipid inclusions merged to form larger droplets and in some cases a single large droplet. Other cells maintained the smaller inclusions without change. These droplets stained positively with the lipid stain Oil Red O [Oil Red O 0.5% in triethylphosphate 60% in $H_2O$].

Preadipocytes derived from the float and the pellet were found to differentiate at different frequencies. Differentiation could be increased by cloning the preadipocytes and other connective tissue cells derived from the pellet and choosing very high responders. When primary cells were cloned in 98 well plates, 8.0% of the clones were inducible with dexamethasone and isobutylmethylxanthine to the 100% level of adipocyte expression, 0.6% failed to express when induced and the remainder were inducible by exogenous chemical induction with dexamethasone and isobutylmethylxanthine to levels between 10 and 75%.

Other experiments indicate the preadipocyte derived from the float gives very high response without cloning. As is shown in Example 4 below, preadipocytes derived from the float are greater than 90% inducible in tissue equivalents both with and without exogenous chemical induction, i.e., they give virtually the same response in the lattice with or without chemical induction.

## Example 4:

### Differentiation of Precursor Cells With and Without Chemical Induction

Cells were isolated from human periadrenal fat obtained from a biopsy by collagenase digestion and preadipocytes cultured and passed as described in Examples 1 and 2 above. The preadipocytes were either cultured in TC flasks as described in Example 2 above or a tissue precursor mixture containing the predipocytes was constituted and cast as a tissue equivalent as described in the Patents and in Example 3 above. Non-cloned preadipocytes were prepared from the pellet as described in Example 2 above and included in tissue equivalents.

These tissue equivalents were used to follow differentiation of the preadipocytes into adipocytes both with and without exogenous chemical induction with dexamethasone and isobutylmethylzanthine. The following results were obtained:

Chemical induction at 48 hours after casting.

Control tissue equivalent: 39% converted to adipocytes.

Induced tissue equivalent: in the lattice 54% converted to adipocytes.

Chemical induction at 5 days after casting.

Control tissue equivalent: 24% converted to adipocytes.

Induced tissue equivalent: 45% converted to adipocytes.

A second experiment using preadipocytes prepared from a different biopsy but otherwise in accordance with Examples 1 and 2 above gave the following results: all induced at 48 hours.

Preadipocytes from the pellet: control 33% induced 70%

Preadipocytes from the float: control 90% induced 93% Dermal fibroblasts: control 18% induced 9%

It is understood that the examples and embodiments described herein are for illustrative purposes only, and that various modifications or changes in light thereof that will be suggested to persons skilled in the art are to be included in the spirit and purview of this application and the scope of the approved claims.

## Claims

1. A method of producing a tissue equivalent comprising:

a. constituting a tissue precursor mixture, the mixture comprising a collagen solution, at least one contractile agent and at least one type of progenitor cell, under conditions to form a gel having the contractile agent and progenitor cells dispersed within the gel; and then

b. maintaining the gel prepared in step (a) under conditions which permit (i) the contraction of the gel to form a tissue equivalent and (ii) the differentiation without exogenous chemical induction of the progenitor cells.

2. A tissue equivalent formed according to the method of claim 1.

3. The method of claim 1, wherein the progenitor cells are preadipocytes, undifferentiated embryonic cells, undifferentiated fetal cells, tissue cells dedifferentiated during cell culture, or organ cells dedifferentiated during cell culture.

4. The method of claim 1, wherein the tissue precursor mixture further comprises non-inducible fibroblasts.

5. A method of augmenting tissue in a living host comprising:

a. administering at the site to be augmented a tissue precursor mixture, the mixture comprising a collagen solution, at least one contractile agent and at least one type of progenitor cell, under conditions to form a gel having the contractile agent and progenitor cells dispersed within the gel mixture.

6. A method of producing a tissue equivalent comprising:

a. constituting a tissue precursor mixture, the mixture comprising a collagen solution, at least one contractile agent and preadipocytes, under conditions to form a gel having the contractile agent and preadipocytes dispersed within the gel; and then

b. maintaining the gel mixture prepared in step (a) under conditions which permit (i) the contraction of the gel mixture to form a tissue equivalent, and (ii) the differentiation without exogenous chemical induction of the preadipocytes into adipocytes.

7. A tissue equivalent formed according to the method of claim 6.

8. A method of augmenting tissue in a living host comprising:

a. administering at the site to be augmented a tissue precursor mixture, the mixture comprising collagen, at least one contractile agent and preadipocytes, under conditions to form at the site a gel having the contractile agent and preadipocytes dispersed within the gel mixture.

9. A method of augmenting tissue in a living host comprising:

a. implanting a tissue equivalent formed according to the method of claim 2 at the site to be augmented.

10. A method of augmenting tissue in a living host comprising:

a. implanting a tissue equivalent formed according to the method of claim 6 at the site to be augmented.

11. A method of differentiating progenitor cells comprising:

a. constituting a mixture, the mixture comprising a collagen solution, at least one contractile agent and at least one type of progenitor cell, under conditions to form a gel having the contractile agent and progenitor cells dispersed within the gel mixture; and then

b. maintaining the gel mixture prepared in step (a) under conditions which permit (i) the contraction of the gel mixture, and (ii) differentiation, without exogenous chemical induction, of the progenitor cells.

12. The method of claim 11, wherein the progenitor cells are preadipocytes, undifferentiated embryonic cells, undifferentiated fetal cells, tissue cells dedifferentiated during cell culture, or organ cells dedifferentiated during cell culture.

European Patent
Office

# EUROPEAN SEARCH REPORT

Application Number

EP 89 30 9972

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | WO-A-8 001 350 (MASSACHUSETTS INSTITUTE OF TECHNOLOGY) * Page 4, lines 31-33; page 6, lines 3-14 * | 1-4 | A 61 L 27/00 A 61 L 15/16 C 12 N 5/00 |
| D,A | WO-A-8 602 273 (E. BELL) * Page 4, lines 14-31; page 5, lines 1-12 * & US-A-46 04 346 | 1-4 | |
| A | EP-A-0 242 270 (INSTITUT MERIEUX S.A.) * Page 3, lines 23-29; example 2 * | 1-2,4 | |
| A | WO-A-8 301 384 (MASSACHUSETTS INSTITUTE OF TECHNOLOGY) * Page 26, lines 2-29 * | 1 | |

| | |
|---|---|
| | TECHNICAL FIELDS SEARCHED (Int. Cl.5) |
| | A 61 L |

The present search report has been drawn up for all claims

| Place of search | Date of completion of the search | Examiner |
|---|---|---|
| THE HAGUE | 22-11-1989 | ESPINOSA Y CARRETERO M. |

EPO FORM 1503 03.82 (P0401)